# EUROPEAN PATENT SPECIFICATION

(11) **EP 2 185 085 B1**
(45) Date of publication and mention of the grant of the patent: **28.10.2015**
(21) Application number: 08788340.1
(22) Date of filing: 14.08.2008
(51) Int. Cl.: A61B 17/42

(54) **APPARATUS FOR CONDUCTING AN EPISIOTOMY**
GERÄT ZUR DURCHFÜHRUNG EINER EPISIOTOMIE
APPAREIL POUR EFFECTUER UNE ÉPISIOTOMIE

(30) Priority: 15.08.2007 GB 0715865
(43) Date of publication of application: 19.05.2010
(73) Proprietor: Plymouth Hospitals NHS Trust, Plymouth PL6 8DH (GB)
(72) Inventor: KAPOOR, Dharmesh, Plymouth PL6 8DH (GB)
(74) Representative: Akers, Noel James
(86) International application number: PCT/GB2008/002771
(87) International publication number: WO 2009/022149

(56) References cited:
- US-A- 2 329 264
- US-A- 2 443 439
- US-A- 5 843 085
- US-A1- 2004 220 579
- US-A1- 2008 097 472

## Description

The present invention relates to a device for conducting an episiotomy and to a method of using the same.

Faecal/anal incontinence is believed to affect from 5 to 10% of the female adult population. One of the major causes of faecal incontinence in women results from childbirth, and arises from damage caused to the tissue extending between the vaginal opening and the anus (perineum), typically due a tear in the perineum arising during the actual birth of the child. Such a tear extending from the vaginal opening can result in damage and long term injury to the anal sphincter muscle, in turn giving rise to the aforementioned incontinence.

Episiotomy is a procedure commonly carried out by a surgeon or midwife during the vaginal birth of a baby. During the procedure, a cut is made into the tissue surrounding the vaginal opening, in order to enlarge the opening and allow the baby to be more easily delivered. It is common practice to administer an episiotomy where there is a high risk that the normal birthing procedure will cause the tissue surrounding the vaginal opening to tear. By making an incision, the object of the episiotomy is to reduce the tissue damage that may result from allowing the tissue to tear naturally. In addition, the damage to the tissue resulting from the incision is more easily repaired, for example by suturing, than tissue damage resulting from a natural tear. Finally, and perhaps most importantly, making an incision in the tissue surrounding the vaginal opening before tearing commences allows the direction of the resulting tear to be controlled, avoiding substantial damage to the surrounding tissue that may be very difficult or impossible to repair.

In general, one of two types of incision are made to the subject during the episiotomy procedure. A midline incision is an incision made along the perineum from the vaginal opening in the direction of the anus. While this form of incision is widely practised, it is associated by the risk that the incision extends during the birthing procedure due to dilation of the vaginal opening and damages the tissue surrounding the anus, in particular the anal sphincter muscle. This can in turn lead to the long term anal incontinence discussed above. As an alternative, a lateral incision may be made, in which case a cut is made in the tissue surrounding the vaginal opening in a lateral direction away from the medial line between the vaginal opening and the anus. Studies have indicated that the risk of sustaining a third degree tear in the tissue decreases by 50% for every increment of 6° the incision is moved away from the perineal midline. Accordingly, there is strong evidence in support of the use of lateral incisions in the episiotomy procedures.

In practice, it is a relatively simple matter for the medical practitioner (accoucheur) delivering the baby to mark up and/or make an incision along the perineal midline by eye, without the aid of instruments. However, this is not the case when the practitioner is wishing to make a lateral incision. Studies have shown that up to 23% of midwives and 1 % of doctors significantly over estimate the angle of a lateral episiotomy incision, leading to the resulting incision being too close to the perineal midline, with the attendant increased risks of a third degree tear and damage to the anal sphincter muscle.

As the birthing procedure proceeds and the head of the baby distends the vaginal opening of the mother, the tissue surrounding the vaginal opening is displaced. This in turn reduces the accuracy of the lateral incision. As a result, an episiotomy conducted after dilation of the vaginal opening has begun, while appearing at the time of making the incision to be at an appropriate lateral angle, can be found to be close to the perineal midline when the patient is relaxed after childbirth.

A range of devices and apparatus are available to the medical practitioner for use in the episiotomy procedure.

Thus, GB 638,892 discloses an early proposal for an aid for carrying out an episiotomy, the aid comprising an elongated scalpel and suture guide, a shielding element, and means for drawing and releasing sutures after their insertion. The device does not assist the medical practitioner in selecting the angle for the episiotomy cut.

Episiotomy scissors are disclosed and shown in US 2,568,234. Similarly, a surgical cutting instrument for use in an episiotomy is described and shown in WO 00/57798.

Surgical guides to assist surgeons and the like in making incisions in subjects are known. For example, US 5,843,085 discloses an osteotomy guide for assisting in conducting an osteotomy procedure. In particular, the guide has first and second slots for guiding a saw, the slots converging at an apex. The guide comprises an array of holes to indicate the position of bone pins to fix the guide in place during use. An incision template for establishing percutaneous intercostals access to the heart of a patient is disclosed in US 2003/0051362 A1. Finally, a guide for locating the incision to be made in a hip replacement procedure is disclosed in US 2004/0220579 A1.

US 2,329,264 discloses a surgical instrument, in particular an episiotome.

Currently, there is nothing to assist a medical practitioner in positioning the incision in an episiotomy procedure. Accordingly, there is a need for a device to aid the medical practitioner in carrying out an episiotomy, in particular in aiding the practitioner to perform an accurate medial incision. It would be particularly advantageous if the device could be as simple as possible to use, while being a simple and low cost item to manufacture.

In a first aspect, the present invention provides an episiotomy guide for use in conducting an episiotomy on a subject, the guide comprising:
a guide means for aligning the guide relative to a line between the vaginal opening and the anus of the subject; characterised by further comprising:
a guide surface rigidly disposed relative to the guide means so as to extend laterally at an angle from the vaginal opening when the guide means is so aligned, the guide surface extending relative to the guide means appropriate for indicating the angle of the line of a lateral episiotomy incision.

The guide of the present invention comprises a guide means. The guide means is used to align the guide between the vaginal opening and the anus of the subject, in particular along the perineal midline. The guide means may comprise any suitable means to provide an indication to the medical practitioner that the guide is properly aligned on the subject. The guide means is preferably such that the means itself may be aligned so as to extend between the vaginal opening and the anus. The guide means may comprise, for example one or marks or lines imprinted, scored or embossed on the surface of the guide. Alternatively, the guide means may comprise one or more slits or slots in the guide, most preferably one or more slits or slots that may be aligned to extend between the vaginal opening and the anus of the subject. In an alternative arrangement, the guide means may comprise a first guide member which, when the guide is in use, extends between the vaginal opening and the anus of the subject. The guide member preferably comprises a surface, most preferably an edge surface that provides the user with an indication of the alignment of the guide, in particular when the guide member is disposed to extend directly between the vaginal opening and the anus, ensures that the guide is properly aligned on the subject.

The guide further comprises a guide surface which, in use when the guide is properly aligned on the subject, extends laterally from the vaginal opening. The guide surface provides the medical practitioner with a clear indication of both the position and orientation of the incision to be made during the episiotomy procedure. The guide surface provides a means for guiding the scissors, or other cutting implement being used to make the episiotomy incision, or for guiding a pen or the like used to mark the skin of the subject to provide a mark for the making of an incision later in the birthing procedure. The guide surface may be formed by a slit or slot in the guide, in which case the tip of a pair of surgical scissors or a marker may be run along the length of the slit or slot to form the incision or the mark, guided by the edge surfaces of the slit or slot. In such a case, the slit or slot is of a width that allows sufficient freedom of movement for the incision or mark to be properly formed, while still acting as an accurate guide to the ensure the eventual incision is at the appropriate angle relative to the line between the vaginal opening and the anus of the subject. Alternatively, the guide surface may be provided by the edge surface of a portion of the guide, in which case the tip of the scissors or marker may be run along the edge surface. In one preferred arrangement, the guide surface is provided on a second guide member, most preferably an edge surface of the guide member.

The guide surface is rigidly disposed relative to the guide means, to ensure that the medical practitioner makes the mark or incision during the episiotomy at the correct position and orientation. In one preferred embodiment, the guide is formed with a unitary construction, that is as a single component or a plurality of components secured together such that the guide surface and the guide means are fixed relative to one another. In an alternative arrangement, the guide may be formed such that the guide surface may be moved relative to the guide means and provided with means to secure, most preferably lock, the guide surface and guide means into a fixed orientation with respect to one another.

The guide may comprise a single guide surface, extending to one side of the guide means. Depending upon the arrangement of the guide, this may limit the use of the guide to forming an incision on the one side of the vaginal opening. Alternatively, the guide may comprise a guide surface extending on each side of the guide means, such that when the guide is in place on the subject, the guide surfaces provide an indication for the medical practitioner on both sides of the vaginal opening, allowing a choice to be made as to which side of the vaginal opening the incision is to be made. In such a case, the guide surfaces on opposing sides of the guide means may extend at the same or different angles to the guide means.

The guide may comprise one or more guide surfaces extending on each side of the guide means, to provide a range of options for the episiotomy incision during use.

In one preferred embodiment, the guide comprises a first elongate guide member bearing the guide means, in particular provided by an edge surface of the first guide member, and a second elongate guide member bearing the guide surface, in particular provided by an edge surface of the second guide member. In such an arrangement, the first and second guide members may be connected at one end, for example extending in a V-shape. The connection is preferably a rigid connection, for example with the guide being formed as a single piece. Alternatively, the first and second guide members may be pivotable about the connection, provided that the angle between the guide means and the guide surface may be fixed before the guide is used.

As noted above, the guide may comprise two second guide members, extending laterally on opposing sides of the first guide member. In this way, in use, a second guide member extends to each side of the vaginal opening, allowing the medical practitioner to choose on which side to make the incision without the need to remove or replace the guide. In a further embodiment, the guide may comprise two or more second guide members extending laterally from one or both sides of the first guide member.

The guide may be formed of any suitable material, for example a metal such as stainless steel, or from plastic, such as polycarbonate. It is an advantage of the guide of the present invention that it may be formed using simple manufacturing procedures, for example stamping or injection moulding. The guide may be formed as a reusable item, in which case the material of construction should be able to withstand the conditions of sterilisation. Alternatively, the guide may be formed as a disposable item for single use.

The guide may be any suitable size, provided that the guide is of a sufficient size to provide accurate guidance to the medical practitioner, while still being accommodated between the legs and thighs of the subject.

The guide surface is disposed at an angle relative to the guide means such that an accurate indication of the position and orientation of a lateral incision in the tissue surrounding the vaginal opening is provided. The angle is preferably a minimum of 25°, more preferably a minimum of 30°. It is preferred that the lateral incision is made at an angle of at least 45° to the perineal midline and therefore the guide surface and guide means are preferably oriented at this angle. The angle between the guide surface and the guide means is preferably up to 80°, more preferably up to 70°. A particularly preferred embodiment of the guide has the guide surface extending at an angle of from 45 to 70° to the guide means.

The guide may be provided with means for temporarily attaching the guide to the tissue surrounding the vaginal opening. In particular, the guide may comprise a clip, for example a spring clip, or the like for engaging with the tissue of the subject. The means should be such that the guide is held in place while the medical practitioner forms the mark or incision on the patient, but should not damage the tissue of the subject while the guide is being held in place. Preferably, the attachment means are disposed to attach the guide to the perineum of the subject.

In a further aspect, the present invention provides the use of a guide as hereinbefore described for carrying out an episiotomy.

In still a further aspect, the present invention provides a method of carrying out an episiotomy on a subject, the method comprising placing a guide means extending between the vaginal opening and the anus of the subject, the guide means having a guide surface rigidly disposed with respect thereto, the guide surface extending laterally at an angle from the vaginal opening; and
using the guide surface as a guide for making an incision extending from the vaginal opening at the said angle.

In the method of the present invention, the guide may be used to prepare a mark on the skin of the subject, for example using a sterile marker pen. Such pens are well known in the art and commercially available. The guide may then be removed and an incision made along the line of the mark at a later stage in the birthing procedure, if desired or necessary. In this respect, the guide may be used to mark the subject at an early stage in the birthing procedure, in particular before dilation of the vaginal opening has begun or is significant. In this way, the eventual incision may be made in the tissue with the medical practitioner being sure that the angle of the incision will be correct and sufficiently removed from the perineal midline.

Alternatively, the guide may be used directly in the making of the incision, with the scalpel or other cutting device being operated along the guide surface.

As discussed above, the guide is preferably attached to the tissue surrounding the vaginal opening, in particular the perineum, while the guide is in use.

The angles at which the mark or incision may be made during the procedure are as described hereinbefore.

An embodiment of the guide of the present invention will now be described, by way of example only, having reference to the accompanying figures, in which:
Figure 1 is a plan view of a guide according.to the present invention; and
Figure 2 is a side view of the guide of Figure 1 along the line II-II.

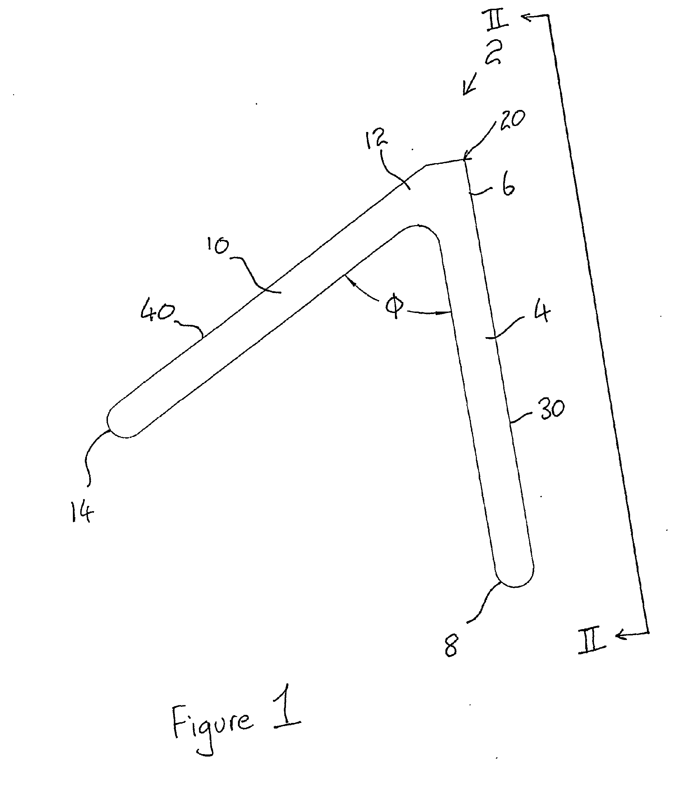

Referring to Figure 1, there is shown in plan view a guide, generally indicated as 2. The guide 2 comprises a first elongate guide member 4 having a first end portion 6 and a rounded second end portion 8. The first guide member 4 is of sufficient length to allow the guide to be aligned with the perineal midline. If desired, the first guide member 4 may be long enough to extend from the vaginal opening to the anus. However, this is not required, provided the first guide member can provide an accurate indication of the aforementioned alignment.

A second elongate guide member 10 has a first end portion 12 connected to the first end portion 6 of the first guide member 4, such that the second guide member 10 extends from the first guide member 4 at an angle Φ, as shown in Figure 1. The second guide member 10 comprises a second end portion 14 having a rounded end. The second guide member 10 is of sufficient length to provide an accurate guide for the incision to be made during the episiotomy. The second guide member 10 may substantially the same length as the first guide member, as shown in the figures, or may be longer or shorter, as required.

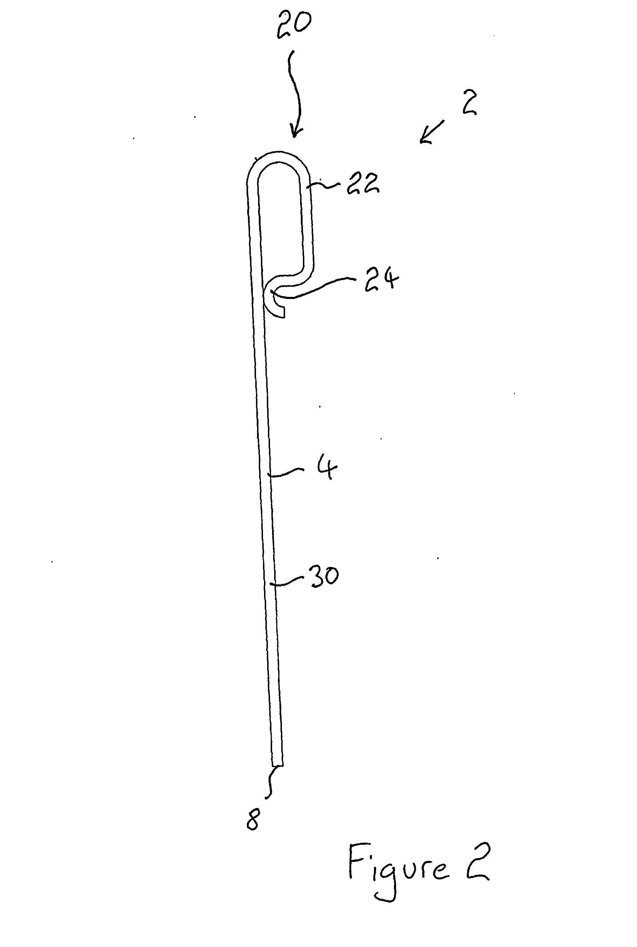

A clip portion 20 extends from the first end portion 6 of the first guide member 4. The clip portion 20 comprises a curved flexible clip arm 22 overlying one surface of the clip portion 20, with the end portion 24 of the clip arm 22 being biased into contact with the clip portion 20. This is shown more clearly in Figure 2. As an alternative to the clip portion shown in Figure 2, the guide may comprise a spring clip.

A guide means is formed by the first guide member 4. A line (not shown) may be formed along the length of the first guide member 4, to aid in alignment of the guide when in use. Alternatively, if the first guide member 4 is narrow enough, the guide member itself may act as the guide means. As a further alternative, the first guide member 4 may be arranged so that the outer edge surface 30 of the member may act as the guide means for the medical practitioner.

As shown in Figure 1, the second guide member 10 extends from the first guide member 4 at an angle of approximately 60°. The guide 2 may be prepared with other values of the angle Φ, as described hereinbefore. It is an advantage of the guide of the present invention, in particular of the embodiment shown in the figures, that a series of guides may be provided having the same general configuration and mode of use, but with different angles Φ. In this way, the medical practitioner may quickly and easily select the appropriate guide according to the angle of the episiotomy incision it is determined is to be made.

The second guide member 10 has an outer edge surface 40, which provides a guide surface for performing an episiotomy.

In use, the guide 2 is attached by means of the clip portion 20 to the perineal tissue of the vaginal opening such that the first guide member 4 extends along the perineal midline of the subject. Once secured in this position, the second guide member 10 extends at the angle Φ to the first guide member 4. As shown in Figure 1, the second guide member 10 will, in use extend to the left of the vaginal opening. It will be appreciated that the second guide member 10 may also extend to the opposite side (that is the right side as viewed in Figure 1).

In an alternative arrangement, the guide 2 may be provided with a pair of second guide members 10, each extending from opposite sides of the first guide member 4. In this way, the medical practitioner may elect which side to make the episiotomy incision without having to change or replace the guide 2 during the birth. The two opposing second guide members 10 may extend at different angles Φ to the first guide member, although it may be more convenient to have the angle Φ the same on each side.

Once the guide 2 is secured to the subject, the edge surface 40 of the second guide member 10 is used as a guide in the episiotomy. The edge surface 40 may be used to guide a sterile marker pen to form a mark on the skin of the subject, which remains when the guide is removed. The mark is then subsequently used during the birthing procedure to guide the making of the episiotomy incision. In this way, the correct position and orientation of the incision may be determined at an early stage in the birth, for example when the medical practitioner has more time and the tissue around the vaginal opening is not distorted by dilation of the opening.

Alternatively, the edge surface 40 may be used directly to guide a scalpel or the like in making an incision. The guide 2 is then removed and the birth continued.

The guide members 4, 10 of the device shown in the figures are sufficiently flexible for them to adapt to the general form of the tissue around the vaginal opening. However, the guide members should be sufficiently stiff and rigid that the angle Φ is maintained in use. Should it be required, the guide 2 may comprise a support member (not shown for clarity) extending from the second end portion 8 of the first guide member 4 to the second end portion 14 of the second guide member 10.

It will also be understood that the first and second guide members 4, 10 may be joined by two or more support members extending between them, or may be the edge portions of a substantially triangular guide member.

## Claims

1. An episiotomy guide (2) for use in conducting an episiotomy on a subject, the guide comprising:
a guide means (4) for indicating alignment of the guide relative to a line between the vaginal opening and the anus of the subject;
**characterised by** further comprising:
a guide surface (10) rigidly disposed relative to the guide means (4) so as to extend laterally at an angle from the vaginal opening when the guide means (4) is so aligned, the guide surface (10) extending relative to the guide means (4) appropriate for indicating the angle of the line of a lateral episiotomy incision.

2. The guide (2) according to claim 1, wherein the guide means (4) itself is adapted to be aligned with the perineal midline extending between the vaginal opening and the anus of the subject.

3. The guide (2) according to claim 1 or 2, wherein the guide means (4) comprises one or more lines or marks for aligning with the perineal midline of the subject and/or wherein the guide means (4) comprises one or more slits or slots for aligning with the perineal midline of the subject.

4. The guide (2) according to any preceding claim, wherein the guide means (4) comprises a first guide member; preferably wherein the first guide member comprises a surface for aligning with the perineal midline of the subject, more preferably wherein the surface is an edge surface of the first guide member.

5. The guide (2) according to any preceding claim, wherein the guide surface (10) comprises a slit or a slot formed in the guide, or wherein the guide surface comprises an edge surface (40) of a portion of the guide, or wherein the guide surface is a surface of a second guide member.

6. The guide (2) according to any preceding claim, wherein the guide means (4) and guide surface (10) are in fixed relationship to one another, preferably wherein the guide is a unitary construction.

7. The guide (2) according to any of claims 1 to 5, wherein the guide surface (10) is moveable with respect to the guide means (4), the guide (2) further comprising means to rigidly locate the guide surface (10) with respect to the guide means (4), preferably wherein the guide (2) comprises means to lock the guide surface (10) with respect to the guide means (4).

8. The guide (2) according to any preceding claim, comprising a plurality of guide surfaces (10), each guide surface arranged to indicate, in use, a different angle for the lateral episiotomy incision; preferably comprising a plurality of guide surfaces (10), the guide surfaces being arranged such that, in use, at least one guide surface (10) extends to each side of the vaginal opening.

9. The guide (2) according to any preceding claim, comprising:
a first elongate guide member bearing the guide means (4); and
a second elongate guide member bearing the guide surface (10);
preferably wherein the guide means (4) is provided by an edge surface of the first elongate guide member and/or wherein the guide surface (10) is provided by an edge surface (40) of the second elongate guide member;
or preferably wherein each of the first and second elongate guide members has an end portion attached to the other of the first and second elongate guide members, especially wherein the first and second elongate guide members extend in a V-shape.

10. The guide (2) according to claim 9, comprising a plurality of second guide members, at least one second guide member extending laterally from each side of the first guide member.

11. The guide (2) according to any preceding claim, being formed from a material that is capable of withstanding repeated sterilisation or wherein the guide is a disposable item for single use.

12. The guide (2) according to any preceding claim, wherein the angle is at least 25°, more preferably wherein the angle is at least 30°, still more preferably wherein the angle is at least 45°; or wherein the angle is no greater than 80°, preferably wherein the angle is no greater than 70°; in particular wherein the angle is from 45 to 70°.

13. The guide (2) according to any preceding claim, further comprising means (20) for removably attaching the guide to the tissue surrounding the vaginal opening of the subject; preferably wherein the said means (20) is for attaching the guide to the perineum of the subject.

14. The guide (2) according to claim 13, wherein the said means is a clip.

15. A kit comprising a plurality of guides (2) according to any of claims 1 to 14, the angle between the guide surface (10) and the guide means (4) being different in at least two of the guides (2).

## Patentansprüche

1. Episiotomieführung (2) für den Gebrauch bei der Durchführung einer Episiotomie an einer Person, wobei die Führung Folgendes umfasst:
ein Führungsmittel (4) zum Anzeigen der Ausrichtung der Führung relativ zu einer Linie zwischen der Vaginalöffnung und dem Anus der Person;
**dadurch gekennzeichnet, dass** es ferner Folgendes umfasst:
eine Führungsfläche (10), die relativ zum Führungsmittel (4) starr angeordnet ist und sich so seitlich in einem Winkel von der Vaginalöffnung erstreckt, wenn das Führungsmittel (4) so ausgerichtet ist, dass sich die Führungsfläche (10) relativ zum Führungsmittel (4) erstreckt und geeignet ist, den Winkel der Linie eines lateralen Episiotomieschnitts anzuzeigen.

2. Führung (2) gemäß Anspruch 1, wobei das Führungsmittel (4) selbst angepasst ist, um an der perinealen Mittellinie, die sich zwischen der Vaginalöffnung und dem Anus der Person erstreckt, ausgerichtet zu werden.

3. Führung (2) gemäß Anspruch 1 oder 2, wobei das Führungsmittel (4) eine oder mehrere Linien oder Markierungen umfasst, um an der perinealen Mittellinie der Person ausgerichtet zu werden, und/oder wobei das Führungsmittel (4) eine oder mehrere Spalten oder Schlitze umfasst, um an der perinealen Mittellinie der Person ausgerichtet zu werden.

4. Führung (2) gemäß einem der vorherigen Ansprüche, wobei das Führungsmittel (4) ein erstes Führungselement umfasst; wobei bevorzugt das erste Führungselement eine Oberfläche zum Ausrichten an der perinealen Mittellinie der Person umfasst, wobei weiter bevorzugt die Oberfläche eine Kantenfläche des ersten Führungselements ist.

5. Führung (2) gemäß einem der vorherigen Ansprüche, wobei die Führungsfläche (10) einen Spalt oder einen Schlitz umfasst, der in der Führung ausgebildet ist, oder wobei die Führungsfläche eine Kantenfläche (40) eines Teils der Führung umfasst oder wobei die Führungsfläche eine Fläche eines zweiten Führungselements ist.

6. Führung (2) gemäß einem der vorherigen Ansprüche, wobei das Führungsmittel (4) und die Führungsfläche (10) sich in einer feststehenden Ausrichtung zueinander befinden, wobei bevorzugt die Führung einen einheitlichen Aufbau aufweist.

7. Führung (2) gemäß einem der Ansprüche 1 bis 5, wobei die Führungsfläche (10) in Bezug auf das Führungsmittel (4) beweglich ist, die Führung (2) ferner ein Mittel zur starren Platzierung der Führungsfläche (10) in Bezug zum Führungsmittel (4) umfasst, wobei bevorzugt die Führung (2) ein Mittel zum Blockieren der Führungsfläche (10) in Bezug auf das Führungsmittel (4) umfasst.

8. Führung (2) gemäß einem der vorherigen Ansprüche, umfassend eine Vielzahl von Führungsflächen (10), wobei jede Führungsfläche angeordnet ist, um bei Gebrauch einen unterschiedlichen Winkel für den lateralen Episiotomieschnitt anzuzeigen; bevorzugt umfassend eine Vielzahl von Führungsflächen (10), wobei die Führungsflächen dergestalt angeordnet sind, dass bei Gebrauch wenigstens eine Führungsfläche (10) sich über jede Seite der Vaginalöffnung erstreckt.

9. Führung (2) gemäß einem der vorherigen Ansprüche, umfassend:
ein erstes längliches Führungselement, das das Führungsmittel (4) trägt; und
ein zweites längliches Führungselement, das die Führungsfläche (10) trägt;
wobei bevorzugt das Führungsmittel (4) durch eine Kantenfläche des ersten länglichen Führungselements gebildet wird und/oder wobei die Führungsfläche (10) durch eine Kantenfläche (40) des zweiten länglichen Führungselements gebildet wird;
oder wobei bevorzugt jedes des ersten und zweiten länglichen Führungselements einen Endabschnitt aufweist, der am anderen ersten oder zweiten Führungselement angebracht ist, wobei sich insbesondere das erste und das zweite längliche Führungselement V-förmig erstrecken.

10. Führung (2) gemäß Anspruch 9, umfassend eine Vielzahl zweiter Führungselemente, wobei wenigstens ein zweites Führungselement sich seitlich von jeder Seite des ersten Führungselements erstreckt.

11. Führung (2) gemäß einem der vorherigen Ansprüche, wobei sie aus einem Material geformt ist, das in der Lage ist, wiederholter Sterilisation standzuhalten, oder wobei die Führung ein Einwegartikel für einmaligen Gebrauch ist.

12. Führung (2) gemäß einem der vorherigen Ansprüche, wobei der Winkel wenigstens 25° beträgt, wobei der Winkel mehr bevorzugt wenigstens 30° beträgt, wobei der Winkel noch mehr bevorzugt wenigstens 45° beträgt oder wobei der Winkel nicht größer als 80° ist, wobei bevorzugt der Winkel nicht größer als 70° ist, wobei der Winkel insbesondere zwischen 45 und 70° beträgt.

13. Führung (2) gemäß einem der vorherigen Ansprüche, ferner umfassend ein Mittel (20), um die Führung entfernbar am Gewebe rund um die Vaginalöffnung der Person anzubringen; wobei das Mittel (20) bevorzugt dazu dient, die Führung am Perineum der Person zu befestigen.

14. Führung (2) gemäß Anspruch 13, wobei das Mittel ein Clip ist.

15. Set umfassend eine Vielzahl von Führungen (2) gemäß den vorherigen Ansprüchen 1 bis 14, wobei der Winkel zwischen der Führungsfläche (10) und dem Führungsmittel (4) bei wenigstens zwei der Führungen (2) unterschiedlich ist.

## Revendications

1. Guide d'épisiotomie (2), destiné à être utilisé pour réaliser une épisiotomie sur un sujet, le guide comprenant :
un moyen de guidage (4) destiné à indiquer l'alignement du guide par rapport à une ligne entre l'ouverture vaginale et l'anus du sujet ;
**caractérisé en ce qu'**il comprend en outre :
une surface de guidage (10) disposée de manière rigide par rapport au moyen de guidage (4) pour s'étendre latéralement à un angle depuis l'ouverture vaginale lorsque le moyen de guidage (4) est aligné, la surface de guidage (10) s'étendant de manière appropriée par rapport au moyen de guidage (4) pour indiquer l'angle de la ligne d'une incision d'épisiotomie latérale.

2. Guide (2) selon la revendication 1, dans lequel le moyen de guidage (4) lui-même est conçu pour être aligné avec la ligne médiane du périnée s'étendant entre l'ouverture vaginale et l'anus du sujet.

3. Guide (2) selon la revendication 1 ou 2, dans lequel le moyen de guidage (4) comprend une ou plusieurs lignes ou marques pour l'alignement avec la ligne médiane du périnée du sujet et/ou dans lequel le moyen de guidage (4) comprend une ou plusieurs fentes ou fissures pour l'alignement avec la ligne médiane du périnée du sujet.

4. Guide (2) selon l'une quelconque des revendications précédentes, dans lequel le moyen de guidage (4) comprend un premier élément de guidage ; le premier élément de guidage comprenant de préférence une surface pour l'alignement avec la ligne médiane du périnée du sujet, la surface étant de préférence une surface de bord du premier élément de guidage.

5. Guide (2) selon l'une quelconque des revendications précédentes, dans lequel la surface de guidage (10) comprend une fente ou une fissure formée dans le guide, ou dans lequel la surface de guidage comprend une surface de bord (40) d'une partie du guide, ou dans lequel la surface de guidage est une surface d'un second élément de guidage.

6. Guide (2) selon l'une quelconque des revendications précédentes, dans lequel le moyen de guidage (4) et la surface de guidage (10) sont fixes l'un par rapport à l'autre, le guide étant de préférence une construction unitaire.

7. Guide (2) selon l'une quelconque des revendications 1 à 5, dans lequel la surface de guidage (10) est mobile par rapport au moyen de guidage (4), le guide (2) comprenant en outre des moyens pour disposer de manière rigide la surface de guidage (10) par rapport au moyen de guidage (4), le guide (2) comprenant de préférence des moyens pour verrouiller la surface de guidage (10) par rapport au moyen de guidage (4).

8. Guide (2) selon l'une quelconque des revendications précédentes, comprenant une pluralité de surfaces de guidage (10), chaque surface de guidage étant agencée pour indiquer, à l'usage, un angle différent pour l'incision d'épisiotomie latérale ; comprenant de préférence une pluralité de surfaces de guidage (10), les surfaces de guidage étant agencées pour qu'à l'usage, au moins une surface de guidage (10) s'étende de chaque côté de l'ouverture vaginale.

9. Guide (2) selon l'une quelconque des revendications précédentes, comprenant :
un premier élément de guidage allongé portant le moyen de guidage (4) ; et
un second élément de guidage allongé portant la surface de guidage (10) ;
le moyen de guidage (4) étant de préférence fourni par une surface de bord du premier élément de guidage allongé et/ou la surface de guidage (10) étant de préférence fournie par une surface de bord (40) du second élément de guidage allongé ;
ou le premier et le second élément de guidage allongé comprenant de préférence chacun une partie d'extrémité attachée à l'autre du premier et du second élément de guidage allongé, le premier et le second élément de guidage allongé s'étendant notamment en une forme en V.

10. Guide (2) selon la revendication 9, comprenant une pluralité de seconds éléments de guidage, au moins un second élément de guidage s'étendant latéralement de chaque côté du premier élément de guidage.

11. Guide (2) selon l'une quelconque des revendications précédentes, étant formé à partir d'un matériau qui est capable de résister à une stérilisation répétée, ou dans lequel le guide est un article jetable à usage unique.

12. Guide (2) selon l'une quelconque des revendications précédentes, dans lequel l'angle est d'au moins 25°, de manière davantage préférée dans lequel l'angle est d'au moins 30°, de manière encore davantage préférée dans lequel l'angle est d'au moins 45° ; ou dans lequel l'angle est inférieur ou égal à 80°, de préférence dans lequel l'angle est inférieur ou égal à 70° ; notamment dans lequel l'angle est de 45 à 70°.

13. Guide (2) selon l'une quelconque des revendications précédentes, comprenant en outre un moyen (20) pour attacher de manière amovible le guide aux tissus entourant l'ouverture vaginale du sujet ; ledit moyen (20) étant de préférence pour attacher le guide au périnée du sujet.

14. Guide (2) selon la revendication 13, dans lequel ledit moyen est un clip.

15. Kit comprenant une pluralité de guides (2) selon l'une quelconque des revendications 1 à 14, l'angle entre la surface de guidage (10) et le moyen de guidage (4) étant différent dans au moins deux des guides (2).
